# EUROPEAN PATENT SPECIFICATION

(11) **EP 2 546 332 B1**
(45) Date of publication and mention of the grant of the patent: **29.06.2016**
(21) Application number: 11173854.8
(22) Date of filing: 13.07.2011
(51) Int. Cl.: C12N 5/00

(54) **Nanotube carrier substrate for primary tissue culture**
Nanorohrträgersubstrat zur Primärgewebekultur
Substrat de support de nanotube pour culture de tissus primaires

(43) Date of publication of application: 16.01.2013
(73) Proprietor: Universität Leipzig, 04109 Leipzig (DE)
(72) Inventor: Mayr, Stefan, 04416 Markkleeberg (DE); Zink, Mareike, 04416 Markkleeberg (DE); Dallacasagrande, Valentina, 29010 Piacenza (IT); Käs, Josef, 04105 Leipzig (DE); Reichenbach, Andreas, 04105 Leipzig (DE)
(74) Representative: Kailuweit & Uhlemann Patentanwälte Partnerschaft mbB

(56) References cited:
- BURNS KEVIN ET AL: "Increased chondrocyte adhesion on nanotubular anodized titanium.", JOURNAL OF BIOMEDICAL MATERIALS RESEARCH. PART A 1 MAR 2009 LNKD- PUBMED:18306319, vol. 88, no. 3, 1 March 2009 (2009-03-01), pages 561-568, XP55015235, ISSN: 1552-4965
- DAS KAKOLI ET AL: "TiO2 nanotubes on Ti: Influence of nanoscale morphology on bone cell-materials interaction.", JOURNAL OF BIOMEDICAL MATERIALS RESEARCH. PART A JUL 2009 LNKD- PUBMED:18496867, vol. 90, no. 1, July 2009 (2009-07), pages 225-237, XP55015197, ISSN: 1552-4965
- KOIZUMI AMANE ET AL: "Organotypic culture of physiologically functional adult mammalian retinas.", PLOS ONE 2007 LNKD- PUBMED:17311097, vol. 2, no. 2, 2007, page E221, XP55015216, ISSN: 1932-6203
- BAUER SEBASTIAN ET AL: "Size selective behavior of mesenchymal stem cells on ZrO(2) and TiO(2) nanotube arrays.", INTEGRATIVE BIOLOGY : QUANTITATIVE BIOSCIENCES FROM NANO TO MACRO SEP 2009 LNKD- PUBMED:20023767, vol. 1, no. 8-9, September 2009 (2009-09), pages 525-532, XP55015225, ISSN: 1757-9708

## Description

The present invention relates to the use of a carrier substrate for primary tissue culture comprising a nanotube array. The invention is applicable in biomedical and biophysical research and clinical applications for *in vitro* culture of explanted tissue.

Organ tissue culture *in vitro* is essential for studying the biology, chemical or physical properties of a living tissue of interest, as well as the effect of drugs and pharmaceuticals on said tissue and the living cells the tissue comprises. It is to be distinguished between *in vitro* culturing of explanted tissue, which is an object of the disclosed invention, and tissue grown *in vitro* to build up new organs for organ transplantations.

Many studies require the culture of an entire tissue freshly explanted from the body (also referred to herein as "organ culture" or "tissue culture", these terms being used synonymously herein) which reflects *in vivo* conditions much better. In organ culture parts of an organ or a whole organ are cultures with the aim to maintain the architecture of the tissue and to direct it towards normal development. Hence, it is essential that the tissue is not disrupted or damaged, which requires careful handling. The culture media used for organ culture are generally the same as those used for cell culture. Therefore the terms "cell culture media" and "tissue culture media" are herein used synonymously.

Organ culture using organs derived from adult animals is challenging and often impossible for explanted mammalian organ tissue due to their greater requirement of oxygen. Greater amounts of serum cannot compensate for this need since it causes toxic effects. Therefore, in the art organ culture is performed usually with embryonic cells that are not differentiated yet rather than performing organ culture with adult organ tissue.

In the art, two main alternatives for cultivation of explanted adult organ tissue are provided. Firstly, instead of cultivating complete tissue samples, cells are extracted from the tissue and cultured without the extracellular matrix surrounding the cells in three-dimensional cell culture (Desai 1999, Eschbach 2005, Giselbrecht 2006, Kunz-Schughart 2004, Leclerc 2003, Snyder 2001, Yamauchi 2003). However by using these methods, the entire tissue is only represented to a very small extent and as the extracellular matrix is lacking, the properties of the cultivated tissue cells and the *in vivo* behavior differ.

Secondly, the entire explanted organ tissue can be cultivated *in vitro.* For this application, conventional cell culture plastic dishes cannot be employed. Therefore various tissue substrates and alternative carrier substrates or devices were developed that assure continuous feeding of the organ with nutrients and that avoid dissolution of the cultured organ.

Organ culture substrates known in the art are nitrocellulose filters (Germer 1997, Kuhrt 2008), cellulose filters (Caffe 2001), PET membranes such as Millipore Millicell inserts (Ogilvie 1999, Johansson 2005), glass (Xin 2007), perforated grids made of stainless steel (Kaempf 2008) or incorporation into agar gels contained in the cell culture medium (Spencer 2008). Further, known organ cultures are performed as roller cultures (Feigenspan 1993, Rzeczinski 2006) or without a support by fixing the organ between two rings (Kobuch 2008). All substrates except for glass and stainless steel grids are soft and highly susceptible and therefore not applicable for many applications that require mechanically stable substrates. Culturing of adult tissues on robust substrates such as glass or also on stainless steel grids could not be performed for several days yet as the cultured organ dies rapidly due to non-physiological conditions.

Since adult mammalian organ tissue is more challenging to culture compared to embryonic tissue, only short culture periods of up to few days can yet be reached on specific substrates. *In vitro* culturing of a mature retina on a cellulose-based substrate could be performed for up to six days (Koizumi 2007).

US 2008/0176206 A1 disclosed a substrate for *in vitro* culturing of cardiovascular tissue. It is comprised of a co-polymeric foam with a bioabsorbable material. The system disclosed in US 2008/0176206 A1 is specifically designed for the regeneration of cardiovascular tissue.

The tissue culture substrates provided in the art are all based of biocompatible organic materials that can be used only once. Further, tissue culture substrates known in the art are not versatile and applicable for a variety of different tissues. Hence, there is a need for universal tissue culture substrates that can be easily designed and optimized for the requirements of different tissues and that allow an *in vitro* organ culture for several days, especially with explanted adult organ tissue.

In the art, metal oxide based cell culture substrates recently got into focus for different culturing applications such as adherent cell culture. WO 2011/052979 A2 discloses a method for reprogramming somatic cells to induced pluripotent stem cells on a nanotube substrate comprising metal oxides. In the method of WO 2011/052979 A2 transcription factors, such as Oct3/4, Sox2, c-Myc, are introduced into a silicon dioxide layer that is located on the surface of a nanotube substrate thus forcing expression of specific genes in the cultured cells.

It is the object of the invention to provide an improved re-usable tissue culture substrate that can be easily adjusted to the requirements of different adult organ tissue.

The object is solved by the use of a tissue culture vessel that is comprised of an outer vessel and a nanotube carrier substrate located within the outer vessel for *in vitro* culturing of primary tissue, preferably primary mammalian tissue, preferably for organ culture. According to the invention the nanotube carrier substrate comprises a nanotube array, wherein the surface roughness of the nanotube array is 1 nm to 100 nm. The term surface roughness as used herein (and in the art) is defined as the standard deviation of the average nanotube height on the surface of the nanotube array. Preferred nanotube arrays are metal oxide nanotube arrays or carbon nanotube arrays, particularly preferred are metal oxide nanotube arrays.

Another aspect of the invention is the use of a nanotube array for *in vitro* culturing of primary tissue, preferably primary mammalian tissue, preferably for organ culture.

Another aspect of the invention is a method for *in vitro* culturing primary tissue, preferably primary mammalian tissue, wherein
- a nanotube array is arranged essentially horizontal inside an outer cell culture vessel, so that openings of the nanotubes point at least in upward direction,
- the nanotube array is contacted with cell culture medium,
- an isolated primary tissue sample is placed on top-side on said nanotube array.

With a essentially horizontal arrangement of the nanotube array inside the outer cell culture vessel is meant herein that in relation to the bottom of the outer cell culture vessel the array of adjacent nanotubes is arranged horizontally (in a range of 170° to 190°, preferably ± 5°), which leads to an essentially vertical (in a range of 80° to 100° preferably 90° ± 5°) arrangement of individual nanotubes with respect to the bottom of the outer cell culture vessel.

The inventors have found that explanted primary adult tissue samples can be successfully cultivated *in vitro* on a titanium dioxide nanotube array for at least 14 days. The explanted tissue samples were therefore placed onto the titanium dioxide nanotube array with cell culture medium fed from the side of the nanotube substrate or, alternatively, from the bottom through the nanotube substrate. Because the nanotube array is wettable by cell culture medium, that is brought in contact with the nanotube array, the latter is drawn into the nanotubes by capillary forces thereby creating a thin film of cell culture medium on the surface of the nanotube array (that is to be distinguished from the bulk liquid of cell culture medium that is filled into the cell culture vessel). The explanted organ itself was not in contact with the cell culture medium bulk liquid but only with the nanotube substrate. The supply of nutrition from the cell culture medium is surprisingly ensured only by the thin film of cell culture medium on the nanotube substrate surface that is continuously restored (by drawing fresh medium onto the substrate by means of capillary forces).

By providing the possibility for culturing explanted organ tissue without direct contact to the bulk liquid of cell culture medium the dissolution of the tissue by cells migrating from the tissue to the liquid or along the nanotube substrate surface is minimized. In-growth of cells from the organ tissue into the nanotube array can be minimized. Thus, with the invention it is possible to provide a tissue culture substrate that allows removal of the cultured organ tissue from the substrate surface without damaging the sensitive tissue structure. However, for some applications it is desired to provide a tissue culture substrate that allows adhesion of the cultured organ tissue, e.g. for measurement of physical parameters of the tissue. This object can also be solved by the invention as by variation of tube diameter or by coating of the nanotube substrate with different bioactive substances to increase tissue adhesion to the nanotube surface.

Advantageously apart from the improved nutrient supply of the organ tissue ensured by the nanotube substrate, the substrate exhibits high mechanical stability (and is thereby easy to handle), is sterilizable and thus reusable for further culturing applications. With methods for varying the properties of nanotube arrays being well known in the art, it is possible to adjust the nanotube substrate properties such as tube diameter, surface roughness and wall thickness easily for any type of organ tissue to be cultured.

Nanotube arrays and methods for their production are known in the art (Roy 2011). A nanotube array comprises a multitude of nanotubes arranged in parallel being adjacent to one another (adjacent individual nanotubes are in contact with each other). The properties of nanotube arrays, such as tube diameter, surface roughness, wall thickness, tube length etc. can be influenced by variation of the processing conditions.

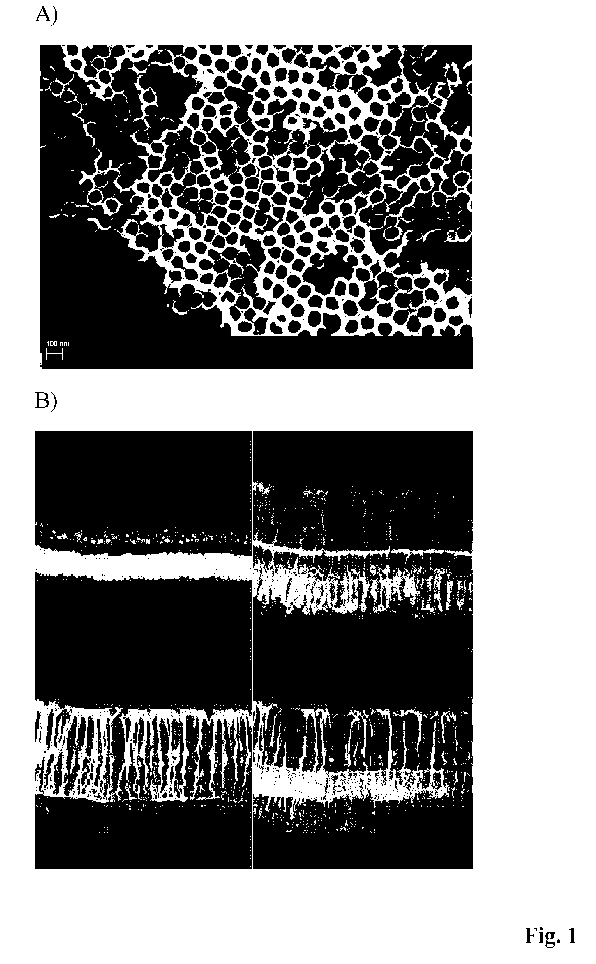

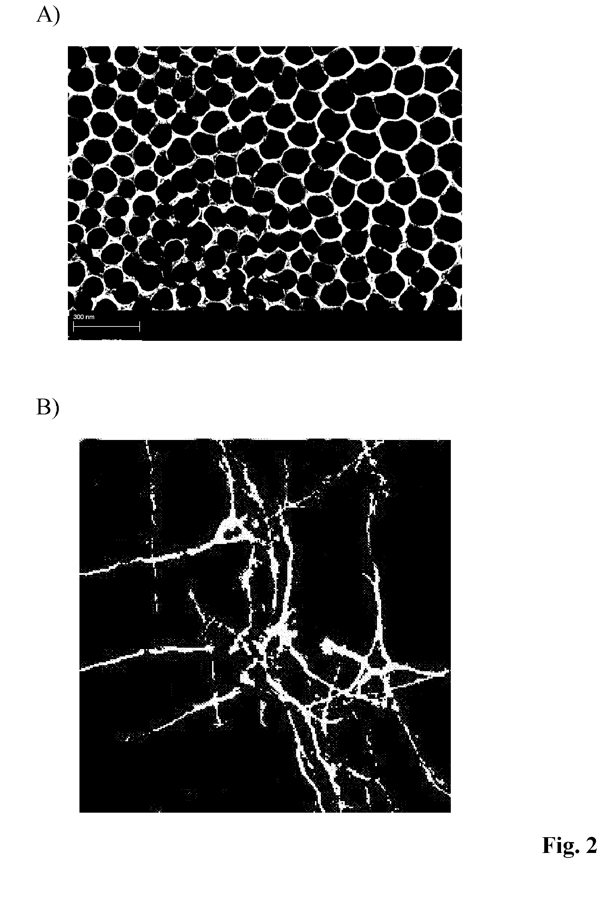

In the invention, metal oxide nanotube arrays or carbon nanotube arrays are preferred. Metal oxide nanotube arrays are commonly formed by electrochemical anodisation of a metallic substrate (e.g. titanium) under a specific set of environmental conditions yielding self aligned nanotube layers of the respective metal oxide (herein referred to as "metal oxide nanotube array", Fig. 1A, 2A). In the invention, nanotube arrays of metal oxides selected from titanium, zirconium, hafnium, silicon, aluminum, gold, silver, copper, platinum, vanadium, palladium, and niobium, as well as their alloys are preferred, particularly preferred are titanium oxide nanotube arrays, particularly preferred are titanium dioxide nanotube arrays. These nanotubes can reside in a crystalline or amorphous structure, which is particularly preferred.

The average inner diameter of the nanotubes contained in the nanotube array is preferably 10 - 500 nm, particularly preferred 20 - 150 nm. The average tube length in the nanotube array is preferably 100 nm - 500 µm. The surface roughness of the nanotube array is preferably 1 nm to 100 nm. Nanotube arrays with a surface roughness of 5 - 100 nm are preferred for cultivation of retinal tissue. Nanotube arrays with a low surface roughness from 1 nm to 10 nm are particularly preferred for cultivation of brain tissue. However, the diameter of the nanotubes mainly influences adhesion and viability of the tissue, whereas surface roughness also influences adhesion, but viability to a smaller extent.

The wall thickness of an individual nanotube contained in the nanotube array is preferably 5 - 45 nm.

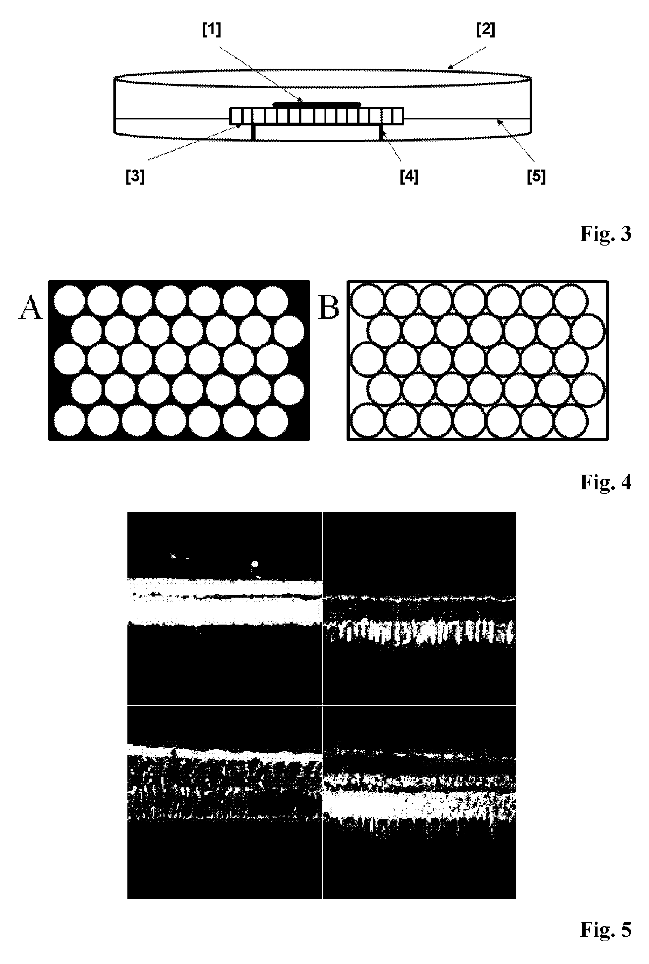

After production of the nanotube array, deposits from the production procedure (debris) can be removed from the nanotube surface of the open nanotube ends. Such debris can smoothen out the surface of the tube array by filling gaps between individual tubes (particularly in the triple point between three tubes) and by partially closing the top openings of a portion of the tubes (such nanotube arrays are herein referred to as "compact nanotube array" and are depicted schematically in Fig. 4A). Debris removal yields porous nanotube structures (Fig. 4B). For such a cleaning procedure, preferably treatments with ultrasound, preferably treatment in an aqueous liquid in a ultrasound bath is employed. For titanium dioxide nanotube arrays, such deposits are titanium hydroxide and/or titanium dioxide which close the gaps between the nanotubes (Fig. 4A) (Wang 2009). By employing the described cleaning treatment, the deposits are removed and the nanotube array becomes more porous (see Fig. 4B). Such cleaning can be employed before the tissue is placed on the nanotube array to change surface properties of the nanotube array and thus, adhesion of the tissue to the nanotube array.

For application in a method or use according to the invention a nanotube array is produced by methods known in the art, a metal oxide nanotube array is preferably produced by anodisation of a metallic substrate. Therefore, a metallic sheet, on which the metal oxide nanotubes array is formed, is used as working electrode.

The nanotube array used in the invention preferably does not contain layers of silicon-containing oxides on its surface (however nanotube arrays of silicone or silicone oxides are preferably employed). Further, the nanotube array preferably does not contain DNA-binding proteins, in particular no transcription factors.

Prior to application in tissue culture the nanotube array is sterilized by methods known in the art, preferably heating, plasma cleaning, ion bombardment, alpha- beta- and gamma-irradiation or rinsing with ethanol (preferred). In a culturing method according to the invention the cultured tissue sample is contacted only with a thin film of cell culture medium on the surface of the nanotube array. In a culturing method according to the invention the cultured tissue sample is preferably not in contact with the cell culture medium bulk liquid. Hence, three general feeding mechanisms are preferred, each one of which uses a differently designed nanotube array substrate:

### 1. Closed bottom nanotube array

This alternative is performed with nanotube arrays being attached to a fully closed solid support material. Hence, the nanotubes have openings at one side and are fully closed on the other side. For culturing tissue in a method according to the invention, a tissue sample is placed on the top side (the side comprising the openings of the nanotubes) of the nanotube array so that the tissue is not in contact with the bulk liquid of the cell culture medium but is only in contact with the thin film of cell culture medium covering the nanotube substrate. To create said thin film of cell culture medium on the surface of the nanotube substrate the nanotube substrate is contacted with the cell culture medium (preferably by contacting the bottom of the nanotube substrate with medium, e.g. by dipping, or by pipetting the medium on the nanotube substrate on the side of the tissue sample). The medium is subsequently drawn into the nanotubes by capillary forces thereby forming a thin layer of cell culture medium on the nanotube substrate. Thereby nutrients from the cell culture medium are continuously fed to the tissue sample via the self-restoring thin film of medium on the top of the nanotube substrate, as well as on all other surfaces of the nanotube array.

Metal oxide nanotube substrates applicable in this feeding mechanism are preferably produced by one of the following alternatives:
After production of a metal oxide nanotube array by methods known in the art, a metal oxide nanotube array is attached to both sides of the initial metallic substrate (top and bottom). A so-formed metal oxide nanotube array is either used directly (with top and bottom nanotubes) or the nanotube array formed at one side is removed, preferably by etching (e.g. by treatment in water-free CH₃OH/Br₂ solution, HF acid, by ion etching or mechanical polishing), yielding a nanotube array being attached to only one side of the initial metallic substrate. The metal oxide nanotube arrays applicable in this feeding mechanism exhibit a high rigidity and hence are easy to handle. The closed bottom metal oxide nanotube array atop a metal substrate is robust and even deformable and allows for attachment of the closed bottom metal oxide nanotube array to external devices, e.g. actuators or motors to determine the effect of deformations of the tissue.

Alternatively the metal oxide nanotube array is removed from the metallic substrate (yielding a free standing nanotube array). Detachment of the metal oxide nanotube array from the initial metallic substrate is preferably employed by drying the metal oxide nanotube array substrate after anodization, preferably for several hours at 20 - 60 °C, which automatically removes the nanotube array from the initial metallic substrate. Additionally, if the nanotubes do not detach from the initial substrate, the nanotube array can be placed in an ultrasonic bath with a liquid, preferably water or ethanol. By that the nanotubes remain closed at the ends which were initially attached to the initial metal substrate, leading to a closed bottom nanotube array.

### 2. Partially closed bottom nanotube array

This alternative is performed with nanotube arrays being attached to a solid, preferably metallic, support material, the solid support material comprising pores (openings in the support material allowing liquid transport through the pores to the other side of the support material), preferably pores of an average diameter of 100 nm - 50 µm, particularly preferred 100 nm - 10 µm. Hence, the nanotubes have openings at one side (top side) and are attached to the porous solid support on the other side (bottom side). For culturing tissue in a method according to the invention, a tissue sample is placed on the top side of the nanotube array. Cell culture medium is fed to the tissue as described in 1 or by contacting the nanotube array with cell culture medium on the bottom of the solid support. If cell culture medium is supplied from the bottom of the solid support, it subsequently perfuses through the pores and draws over the nanotube surface by capillary forces. The medium is subsequently forming a thin layer of cell culture medium on the nanotube substrate.

The partially closed bottom nanotube array allows a more homogenous feeding of the tissue by continuously feeding cell culture medium through the bottom of the nanotube array.

Metal oxide nanotube substrates applicable in this feeding mechanism are preferably produced by one of the following alternatives:
(i) After production of a metal oxide nanotube array by methods known in the art, a metal oxide nanotube array is attached to both sides of the initial metallic substrate (top and bottom). A so-formed metal oxide nanotube array is either used directly (with top and bottom nanotubes) or the nanotube array formed at one side of the metallic substrate is removed, preferably by etching (e.g. by treatment in water-free CH₃OH/Br₂ solution or HF acid), laser structuring, lithography or mechanical drilling yielding a nanotube array being attached to only one side of the initial metallic substrate. Pores are introduced into the metallic substrate by methods known in the art, preferably by means of focused ion beam, laser structuring, laser lithography or mechanical drilling.
(ii) Alternatively, a partially closed bottom nanotube array can be produced by coating a porous metallic substrate (preferably pores of an average diameter of 100 nm - 50 µm, particularly preferred 100 nm - 10 µm, metallic substrates as preferred above) with a metallic layer, preferably titanium, employing physical vapor deposition, sputter deposition or pulsed laser deposition followed by subsequent anodization as described in 1) to yield a metal oxide nanotube array with a porous substrate material on the nanotube bottom.
(iii) The metal oxide nanotube array is removed from the metallic substrate as described in 1), yielding a free standing nanotube array with closed bottom ends of the nanotubes into which pores are introduced by methods known in the art, preferably by means of focused ion beam, laser structuring, lithography or mechanical drilling.
(iv) Free-standing nanotube arrays with nanotubes open on both ends are produced. Here the nanotubes are removed from the initial metallic support material by methods described in 1) which yield nanotubes with top ends open and bottom ends closed. Methods known in the art, preferably treating the nanotube array with ultrasound in aqueous solution or etching the closed nanotube ends are employed to open the nanotube bottom ends. The nanotube array with both nanotube ends open is then attached on top of a porous support material (preferably a metallic support material with pores of an average diameter of 100 nm - 50 µm, particularly preferred 100 nm - 10 µm).

For methods (i) - (iv) etching and ultrasound methods described in 1) can additionally be employed to open a channel all the way from the porous support to the nanotube surface.

Preferably the nanotube array is arranged on a porous support material, preferably a grid, for culturing of a tissue sample to allow easier adjustment of the height of the cell culture medium bulk liquid.

### 3. Free standing open nanotube array

This alternative is performed with nanotube arrays that have openings at both of their ends (top and bottom side) and that are not attached to a solid support at the top and bottom side (named herein "free-standing open nanotubes"). For culturing tissue in a method according to the invention, a tissue sample is placed on the top side of the nanotube array. Cell culture medium is fed to the tissue by contacting the nanotube array with cell culture medium (at the bottom and/or side) of the array (as described in 1 and 2). Preferably the nanotube array is dipped into the cell culture medium, the cell culture medium bulk liquid being in contact with the bottom of the nanotubes, the bulk liquid not fully (preferably not at all) covering the top side of the nanotubes. The cell culture medium is drawn into the nanotubes by capillary forces thereby forming a thin layer of cell culture medium on the surface of the nanotube substrate.

The nanotube arrays applicable in this feeding mechanism exhibit less rigidity than the nanotube array substrates of the previously described feeding mechanisms 1 and 2 but the array is still more rigid than organic substrates. Rigidity of the free standing nanotube array can however be improved by using larger nanotube lengths, preferably more than 100 µm. With free standing nanotube arrays the medium can be fed continuously and homogenously over the entire tissue surface.

Metal oxide nanotube substrates applicable in this feeding mechanism are preferably produced as follows:
After production of a metal oxide nanotube array by methods known in the art, a metal oxide nanotube array is attached to both sides the initial metallic substrate (top and bottom). The nanotube array formed on one side of the metallic substrate is lifted-off from the metallic substrate by ultrasonic treatments or by dissolving the substrate and the nanotubes on the opposite side (as described above), yielding a free-standing open metal oxide nanotube array.

Depending on the application, attachment of the cells of the cultured organ tissue to the nanotube substrate can be advantageous, e.g. when mechanical property measurements of cultured tissue are to be performed. In one embodiment of the invention the surface of the nanotube array is functionalized with bioactive substances, preferably substances that improve attachment of the organ tissue to the nanotube substrate, particularly preferred selected from poly-d-lysin, poly-L-lysin, collagen, fibronectin and laminin.

Vessels applicable for cell and tissue culture are known in the art and are applicable for tissue culture in the invention in combination with a nanotube array as defined above. Preferred are plastic vessels, preferably vessels from polystyrene, in the form of bottles, flaks or dishes. Culture media used in tissue culture in the invention are standard cell culture media known in the art for the cultivation of the respective tissue. In a tissue culture vessel used in the invention the nanotube carrier substrate is preferably arranged horizontally within the outer vessel. Preferably the nanotube carrier substrate is arranged on a porous support material, preferably a grid (preferably a metallic or plastic grid), to allow an easier control of the level of cell culture medium contacting the nanotube carrier substrate.

The invention provides uses and methods for primary tissue culture, particularly preferred organ culture, which is to be distinguished from standard cell culture (where isolated cells are cultured in adherent or suspension culture) and tissue engineering (where cells are cultured on a three-dimensional matrix to differentiate and form a tissue or organ). Tissue to be cultured with the invention is explanted from a subject, preferably a mammal, and subsequently cultured *in vitro.* The invention is preferred for *in vitro* culture of brain, retinal, skin, liver, kidney, lung or heart tissue, preferably brain or retinal tissue. Preferably the cultured tissue is adult tissue, preferably adult mammalian tissue.

For retinal tissue culture the following nanotube array parameters are preferred (individually and in combination with each other): inner tube diameter 20-120 nm (preferably 60 - 100 nm), wall thickness 5-30 nm, surface roughness 5-100 nm. For brain tissue culture the following nanotube array parameters are preferred (individually and in combination with each other): inner tube diameter 10-120 nm, wall thickness 20-40 nm, surface roughness 1-50 nm.

The invention is further illustrated by the following figures and examples without being limited to these.
- Fig. 1: Adult guinea pig retina cultured on a titanium dioxide nanotube array. A) Top view of a titanium dioxide nanotube array (recorded by SEM). The nanotubes exhibit an inner diameter of 80 nm (average) and a wall thickness of 10 - 15 nm. This substrate exhibits a surface roughness of 50 - 100 nm and is applicable for *in vitro* culture of mammalian adult retina tissue. B) Adult guinea pig retina cultured on the nanotube substrate of A) for 7 days. After culture, the retina was removed from the substrate and indirectly immunostained for nuclei (top left), photo receptors (top right), Müller cells (retina glial cells; bottom left). An overlay of the stainings is shown in the image at bottom right side.
- Fig. 2: Adult mouse brain slice cultured on a titanium dioxide nanotube array. A) Top view of a titanium dioxide nanotube array (recorded by SEM). The nanotubes exhibit an inner diameter of 80 nm (average) and a wall thickness of 35 - 40 nm. This substrate exhibits a surface roughness of 5 - 10 nm and is applicable for *in vitro* culture of mammalian adult brain tissue. B) Image of immunostained murine brain slice cultured on the titanium nanotube array according to A) for six days indirectly stained with neurofilament marker SMI32 to image the neurofilaments of the neuronal cells.
- Fig. 3: Illustration of a cell culture vessel including a nanotube carrier substrate in its application. The nanotube carrier substrate (nanotube array, [3]) is located in an outer cell culture dish [2] and (optionally) placed on a grid [4]. Cell culture medium [5] is filled into the cell culture dish to contact the nanotube carrier substrate at the bottom and sides (arrow indicates the height of the cell culture medium inside the cell culture dish). The nanotube carrier substrate sticks out of the cell culture medium bulk liquid and the tissue sample [1] is located on the top side of the nanotube substrate. A thin self-restoring film of cell culture medium is drawn onto the surface of the nanotube carrier substrate thereby feeding the tissue sample with cell culture medium.
- Fig. 4: Schematic drawing of nanotube arrays (top view). A) compact nanotube array. B) porous nanotube array obtainable by ultrasound treatment of a compact nanotube array. Black areas indicate solid material (e.g. titanium dioxide), white areas indicate pores.
- Fig. 5: Confocal laser scanning microscopy image of a freshly extracted guinea pig retina. The retina was immunostained for nuclei (top left), photo receptors (top right), Müller cells (retina glial cells; bottom left). An overlay of the stainings is shown in the image at bottom right side.

### Example 1: Cultivation of an adult guinea pig retina in vitro

Adult guinea pig retina was explanted and cultured on a titanium dioxide nanotube array as shown in Fig. 1A that exhibited a surface roughness of 50-100 nm and an average nanotube diameter of 80 nm. The nanotube substrate was chosen in the form of a closed bottom nanotube array (where the nanotubes were grown on a continuous titanium plate), deposited on a grid inside an outer cell culture vessel (commercially available cell culture dish) as illustrated in Fig. 3. A nanotube substrate with closed tubes on both sides of the titanium plate was used.

Adult guinea pig retina was isolated from eyes of adult guinea pig. After enucleation of the eyes, they were transferred into Ames cell culture medium (Sigma-Aldrich:A1420) without any supplements at 4 °C and were processed under aseptic conditions. The eyes were quickly rinsed in ethanol 70 % (v/v), washed in PBS and kept in the Ames medium without supplements. An incision was made between cornea and sclera to lower down the pressure and remove the lens and the vitreous body. At this point the retina was gently removed from the eye cup and cut in four pieces. The retinal pieces were flat-mounted with the photoreceptor-side down on the nanotube substrate. The nanotube substrate was placed on the grid inside a cell culture dish which was filled with cell culture medium of a height to touch the nanotube substrate bottom and sides. However, the top side of the nanotube substrate and the retina tissue were not covered by culture medium. Nutrient supply from the cell culture medium was assured by perfusion of the medium up the side walls of the nanotube substrate by capillary forces to the bottom side of the tissue sample. The culture medium (Ames Medium, 10 % Horse Serum, 0.1 % Gentamicin) was changed the day after isolation and then every third day. It is known that adult retinal tissue gets destroyed and dies when covered with liquid medium during the duration of *in vitro* culture on different organ culture substrates. Surprisingly, although being cultured on a closed bottom nanotube array fed with cell culture medium by pipetting it to the side ends of the retina instead of perfusion through the nanotubes from the bottom the retina was viable for up to 14 days.

For immunohistochemical staining the isolated and cultured retinas (after 7 days of culture) were fixed after culture in 4% paraformalaldehyde overnight at 4 °C. After washing in PSB the tissues were embedded in PSB contain 3% agarose (Sigma-Aldrich Fluka:05073), and then 50 µm-thick slices were cut with a vibration microtome (HM 650 V; Microm-International, Walldorf, Germany). The slices were incubated in 5% normal goat serum and 0,3% Triton, 1% DMSO in PBS (PSB-TD) for 1 h at 5°C, then washed with PBS-TD. The slices were exposed overnight at 4 °C to monoclonal AntiVimentin Clone 9 (Sigma-Aldrich V6630) and peanut agglutinin (PNA) (Sigma-Aldrich L6135). The day after the slices were washed with PBS-TD for 3 hours on the shaker changing the PBS-TD every hour, then exposed to Cy2-conjugated streptavidin and Cy3-conjugated goat-antimouse (Jackson Immunoresrearch) overnight at 4 °C. The third day the slices were washed with PBS-TD for 1 hour on the shaker, then incubated with TO-PRO-3-iodide (Invitrogen: T3605) for 1 hour at room temperature. The last step is washing the slices with normal PBS and then put them on a coverslip coated with IMMUNOMOUNT. Confocal imaging was carried using an upright laser scanning microscope (Zeiss 510 META, Jena, Germany) equipped with an enterprise laser.

A confocal laser scanning microscopy image of the retina after 7 days of culture is shown in Fig. 1B. The image shows the intact and viable structure of the retina with the healthy Müller cells and photoreceptors. For comparison, Fig. 5 shows a confocal laser scanning microscopy image of a freshly extracted guinea pig retina using identical markers and staining procedure. As can be seen in Fig. 1B an organotypic culture of the adult retina was obtained. The cultured organ tissue could be detached from the substrate for later analysis without disruption.

In another experiment, an adult guinea pig retina was cultured under the same conditions on a nanotube as shown in Fig. 1A, which was coated with poly-d-lysin before the retina was placed on it. Coating of the nanotube array with poly-d-lysin improved physiological conditions and increased the adhesion of the retina to the nanotube substrate.

### Example 2: Cultivation of an adult murine brain slices in vitro

Brain slices from adult mice were cultured on a titanium dioxide nanotube array as shown in Fig. 2A that exhibited a surface roughness of 5-10 nm and an average nanotube diameter of 80 nm. The nanotube substrate was chosen in the form of a closed bottom nanotube array (where the nanotubes were grown on a continuous titanium plate). The nanotube substrate was chosen in the form of a closed bottom nanotube array (where the nanotubes were grown on a continuous titanium plate), deposited on a grid inside an outer cell culture vessel (commercially available cell culture dish) as illustrated in Fig. 3. A nanotube substrate with closed tubes on both sides of the titanium plate was used.

Brain was extracted from adult mice, cut into slices of about 100 - 250 µm thickness and placed onto the top of a titanium dioxide nanotube array as shown in Fig. 2A. The cells were cultured in Hams DMEM medium with L-Glutamin, 24% horse serum, 1% Glucose, 2% HEPES, 0.1% gentamycin (GIBCO) (other conditions being equal as described in example 1). After 6 days of *in vitro* culture the neuronal cells were immunostained with SMI32-Cy3 (neurofilaments) (Fig. 2B). The neurons within the brain slice remained viable and organotypic over the whole culture period.

### Cited non patent literature

Caffe 2001 Caffé AR, Ahuja P, Holmqvist B, Azadi S, Forsell J, Holmqvist I, Söderpalm AK, van Veen T.: J Chem Neuroanat. 2001 Nov;22(4):263-73.
Desai et al. 1999 T. A. Desai, J. Deutsch, D. Motlagh, W. Tan and B. Russell, Biomed. Microdev. 2, 123 (1999)
Eschbach 2005 E. Eschbach, S. S. Chatterjee, M. Nöldner, E. Gottwald, H. Dertinger, K.-F. Weibezahn and G. Knedlitschek, Journal of Cellular Biochemistry 95, 243 (2005)
Feigenspan 1993 Feigenspan A, Bormann J, Wässle H.: Vis Neurosci. 1993 Mar-Apr;10(2):203-17.
Germer 1997 Germer A, Jahnke C, Mack A, Enzmann V, Reichenbach A.: Neuroreport. 1997 Sep 29;8(14):3067-72.
Giselbrecht 2006 S. Giselbrecht, T. Gietzelt, E. Gottwald, C. Trautmann, R. Truckenmüller, K. F. Weibezahn and A. Welle, Biomed. Microdev. 8, 191 (2006
Johansson 2005 Johansson K, Ehinger B.: Vision Res. 2005 Nov;45(25-26):3235-43. Epub 2005 Jul 15.
Kaempf 2008 Kaempf S, Walter P, Salz AK, Thumann G.: J Neurosci Methods. 2008 Aug 15;173(1):47-58. Epub 2008 Jul 15.
Kobuch 2008 Kobuch K, Herrmann WA, Framme C, Sachs HG, Gabel VP, Hillenkamp J.: Exp Eye Res. 2008 Apr;86(4):661-8. Epub 2008 Jan 18.
Koizumi 2007 Koizumi, G. Zeck, Y. Ben, R. H. Masland and T. C. Jacobs, Plos ONE 2, e221 (2007)
Kuhrt 2008 Kuhrt H, Wurm A, Karl A, landiev I, Wiedemann P, Reichenbach A, Bringmann A, Pannicke T.: Int J Dev Neurosci. 2008 Nov;26(7):745-51. Epub 2008 Jul 11.
Kunz-Schughart 2004 L. A. Kunz-Schughart, J. P. Freyer, F. Hofstaedter and R. Ebner, J. Biomol. Screen. 9, 273 (2004)
Leclerc 2003 E. Leclerc, Y. Sakai and T. Fujii, Biomed. Microdev. 5, 209 (2003) Ogilvie 1999 Ogilvie JM, Speck JD, Lett JM, Fleming TT.: J Neurosci Methods. 1999 Feb 1;87(1):57-65.
Roy 2011 Roy P, Berger S, Schmuki P.: Angew Chem Int Ed Engl. 2011 Mar 21;50(13):2904-39. doi: 10.1002/anie.201001374. Epub 2011 Mar 10. Review
Rzeczinski 2006 Rzeczinski S, Victorov IV, Lyjin AA, Aleksandrova OP, Harms C, Kronenberg G, Freyer D, Scheibe F, Priller J, Endres M, Dirnagl U.: Ophthalmic Res. 2006;38(5):263-9. Epub 2006 Sep 15.
Snyder 2001 J. D. Snyder and T. A. Desai, J. Biomater. Sci. Polym Ed. 12, 921 (2001)
Spencer 2008 N. J. Spencer, D. A. Cotanche and C. M. Klapperich, Biomaterials 29, 1028 (2008)
Wang 2009 Chemistry of Materials 21 (7), 1198-1206 (2009)
Xin 2007 Xin H, Yannazzo JA, Duncan RS, Gregg EV, Singh M, Koulen P.: J
Neurosci Methods. 2007 Jan 15;159(1):35-42. Epub 2006 Jul 31. Yamauchi 2003 N. Yamauchi, O. Yamada, T. Takahashi, K. Imai, T. Sato, A. Ito and K. Hashizume, Placenta 24, 258 (2003)

## Claims

1. Use of a nanotube array for *in vitro* culturing of primary tissue, preferably primary mammalian tissue, preferably for organ culture.

2. Use according claim 1, wherein the surface roughness of the nanotube array is 1 nm to 100 nm.

3. Use of a tissue culture vessel comprising an outer vessel and a nanotube carrier substrate located within the outer vessel, wherein the nanotube carrier substrate comprises a nanotube array, wherein the surface roughness of the nanotube array is 1 nm to 100 nm for *in vitro* culturing of primary tissue, preferably primary mammalian tissue, preferably for organ culture.

4. Method for *in vitro* culturing primary tissue, preferably primary mammalian tissue, wherein
- a nanotube array is horizontally arranged inside an outer tissue culture vessel, so that openings of the nanotubes point at least in upward direction,
- the nanotube array is contacted with cell culture medium,
- an isolated primary tissue sample is placed on said nanotube array.

5. Method of claim 4, wherein the upper surface of said nanotube array sticks out of the cell culture medium bulk liquid or is adjusted at the same height of the cell culture medium bulk liquid surface.

6. Use of any of claims 1 to 3 or method of claim 4 wherein the nanotube array is a metal oxide nanotube array or a carbon nanotube array, preferably a metal oxide nanotube array, particularly preferred from nanotubes of oxides of titanium, zirconium, hafnium, silicon, aluminum, gold, silver, copper, platinum, vanadium, palladium, and/or niobium, preferably titanium oxide.

7. Use of any of claims1 to 3 or 6, or method of claim 4 or 6 wherein the inner diameter of the nanotubes is 10 - 500 nm.

8. Use of any one of claims 1 to 3, 6 and 7 or method of any one of claims 4, 6 and 7 wherein the of nanotube array comprises an upper side and a bottom side, the upper side comprising openings of the nanotubes and the bottom side being closed, preferably by a solid support material attached to the nanotube array at the bottom side.

9. Use of any one of claims 1 to 3, 6 and 7 or method of any one of claims 4, 6 and 7 wherein the nanotube array comprises an upper side and a bottom side, the upper side comprising openings of the nanotubes and the bottom side being attached to a solid support material containing pores of a diameter of 100 nm to 50 µm.

10. Use of any one of claims 1 to 3, 6 and 7 or method of any one of claims 4, 6 and 7 wherein the nanotube array comprises an upper side and a bottom side, both of said sides comprising openings of the nanotubes.

11. Use of any one of claims 1 to 3 and 6 to 10 or method of any one of claims 4 and 6 to 10 wherein the nanotube array is obtainable by electrolytic anodisation using a metallic sheet, on which a metal oxide nanotube array is formed, as working electrode.

12. Use of any one of claims 1 to 3 and 6 to 11 or method of any one of claims 4 and 6 to 11 wherein the nanotube array comprises a surface coating comprising at least one bioactive substance, preferably selected from poly-d-lysin, poly-L-lysin, collagen, fibronectin and laminin.

## Patentansprüche

1. Verwendung einer Nanoröhrenanordnung zur *in vitro-*Kultur von Primärgewebe, vorzugsweise Säugetier-Primärgewebe, vorzugsweise für die Organkultur.

2. Verwendung nach Anspruch 1, wobei die Oberflächenrauheit der Nanoröhrenanordnung 1 nm bis 100 nm beträgt.

3. Verwendung eines Gewebekulturgefäßes, umfassend ein äußeres Gefäß und ein Nanoröhren-Trägersubstrat, das im äußeren Gefäß angeordnet ist, wobei das Nanoröhren-Trägersubstrat eine Nanoröhrenanordnung umfasst, wobei die Oberflächenrauheit der Nanoröhrenanordnung 1 nm bis 100 nm beträgt, für die *in vitro*-Kultur von Primärgewebe, vorzugsweise Säugetier-Primärgewebe, vorzugsweise für die Organkultur.

4. Verfahren zur *in vitro*-Kultur von Primärgewebe, vorzugsweise Säugetier-Primärgewebe, wobei
- eine Nanoröhrenanordnung horizontal in einem äußeren Gewebekulturgefäß angeordnet ist, so dass Öffnungen der Nanoröhren wenigstens nach oben zeigen,
- die Nanoröhrenanordnung mit Zellkulturmedium in Kontakt gebracht wird,
- eine isolierte Primärgewebeprobe auf der Nanoröhrenanordnung anbracht wird.

5. Verfahren nach Anspruch 4, wobei die obere Fläche der Nanoröhrenanordnung aus dem Flüssigkeitskörper des Zellkulturmediums hervorsteht oder auf die gleiche Höhe wie die Oberfläche des Flüssigkeitskörpers des Zellkulturmediums eingestellt ist.

6. Verwendung nach einem der Ansprüche 1 bis 3 oder Verfahren nach Anspruch 4, wobei die Nanoröhrenanordnung eine Metalloxidnanoröhren-Anordnung oder eine Kohlenstoffnanoröhren-Anordnung ist, vorzugsweise eine Metalloxidnanoröhren-Anordnung, besonders bevorzugt aus Nanoröhren aus Oxiden von Titan, Zirconium, Hafnium, Silicium, Aluminium, Gold, Silber, Kupfer, Platin, Vanadium, Palladium und/oder Niob, vorzugsweise Titanoxid.

7. Verwendung nach einem der Ansprüche 1 bis 3 oder 6 oder Verfahren nach Anspruch 4 oder 6, wobei der Innendurchmesser der Nanoröhren 10 bis 500 nm beträgt.

8. Verwendung nach einem der Ansprüche 1 bis 3, 6 und 7 oder Verfahren nach einem der Ansprüche 4, 6 und 7, wobei die Nanoröhrenanordnung eine Oberseite und eine Unterseite umfasst, wobei die Oberseite Öffnungen der Nanoröhren umfasst und die Unterseite geschlossen ist, vorzugsweise durch ein festes Auflagematerial, das an der Nanoröhrenanordnung an der Unterseite befestigt ist.

9. Verwendung nach einem der Ansprüche 1 bis 3, 6 und 7 oder Verfahren nach einem der Ansprüche 4, 6 und 7, wobei die Nanoröhrenanordnung eine Oberseite und eine Unterseite umfasst, wobei die Oberseite Öffnungen der Nanoröhren umfasst und die Unterseite an einem festen Auflagematerial befestigt ist, das Poren mit einem Durchmesser von 100 nm bis 50 µm enthält.

10. Verwendung nach einem der Ansprüche 1 bis 3, 6 und 7 oder Verfahren nach einem der Ansprüche 4, 6 und 7, wobei die Nanoröhrenanordnung eine Oberseite und eine Unterseite umfasst, wobei beide Seiten Öffnungen der Nanoröhren umfassen.

11. Verwendung nach einem der Ansprüche 1 bis 3 und 6 bis 10 oder Verfahren nach einem der Ansprüche 4 und 6 bis 10, wobei die Nanoröhrenanordnung durch elektrolytische Anodisierung hergestellt werden kann, wobei eine metallische Folie, auf der eine Metalloxid-Nanoröhrenanordnung gebildet ist, als Arbeitselektrode verwendet wird.

12. Verwendung nach einem der Ansprüche 1 bis 3 und 6 bis 11 oder Verfahren nach einem der Ansprüche 4 und 6 bis 11, wobei die Nanoröhrenanordnung eine Oberflächenbeschichtung umfasst, die wenigstens eine bioaktive Substanz umfasst, die vorzugsweise aus Poly-d-lysin, Poly-L-lysin, Collagen, Fibronectin und Laminin ausgewählt ist.

## Revendications

1. Utilisation d'un réseau de nanotubes pour la culture *in vitro* de tissu primaire, de préférence de tissu primaire mammalien, de préférence pour la culture d'organes.

2. Utilisation selon la revendication 1, dans laquelle la rugosité de surface du réseau de nanotubes est de 1 nm à 100 nm.

3. Utilisation d'un récipient de culture tissulaire, comprenant un récipient extérieur et un substrat de support de nanotubes situé à l'intérieur du récipient extérieur, dans laquelle le substrat de support de nanotubes comprend un réseau de nanotubes, dans laquelle la rugosité de surface du réseau de nanotubes est de 1 nm à 100 nm, pour la culture *in vitro* de tissu primaire, de préférence de tissu primaire mammalien, de préférence pour la culture d'organes.

4. Procédé de culture *in vitro* de tissu primaire, de préférence de tissu primaire mammalien, dans lequel
- un réseau de nanotubes est disposé horizontalement à l'intérieur d'un récipient de culture tissulaire extérieur, de telle manière que les ouvertures des nanotubes pointent au moins dans la direction du haut,
- le réseau de nanotubes est mis en contact avec le milieu de culture cellulaire,
- un échantillon de tissu primaire isolé est placé sur ledit réseau de nanotubes.

5. Procédé selon la revendication 4, dans lequel la surface supérieure dudit réseau de nanotubes dépasse du volume liquide du milieu de culture cellulaire ou est ajustée à la même hauteur que celle de la surface du volume liquide du milieu de culture cellulaire.

6. Utilisation selon l'une quelconque des revendications 1 à 3, ou procédé selon la revendication 4, dans lesquels le réseau de nanotubes est un réseau de nanotubes d'oxyde de métal ou un réseau de nanotubes de carbone, de préférence un réseau de nanotubes d'oxyde de métal, de manière particulièrement préférée de nanotubes d'oxyde de titane, de zirconium, de hafnium, de silicium, d'aluminium, d'or, d'argent, de cuivre, de platine, de vanadium, de palladium, et/ou de niobium, de préférence d'oxyde de titane.

7. Utilisation selon l'une quelconque des revendications 1 à 3 ou 6, ou procédé selon la revendication 4 ou 6, dans lesquels le diamètre intérieur des nanotubes est de 10 à 500 nm.

8. Utilisation selon l'une quelconque des revendications 1 à 3, 6 et 7, ou procédé selon l'une quelconque des revendications 4, 6 et 7, dans lesquels le réseau de nanotubes comprend un côté supérieur et un côté inférieur, le côté supérieur comprenant les ouvertures des nanotubes et le côté inférieur étant fermé, de préférence par un matériau de support solide fixé au réseau de nanotubes au niveau du côté inférieur.

9. Utilisation selon l'une quelconque des revendications 1 à 3, 6 et 7, ou procédé selon l'une quelconque des revendications 4, 6 et 7, dans lesquels le réseau de nanotubes comprend un côté supérieur et un côté inférieur, le côté supérieur comprenant les ouvertures des nanotubes et le côté inférieur étant fixé à un matériau de support solide contenant des pores d'un diamètre de 100 nm à 50 µm.

10. Utilisation selon l'une quelconque des revendications 1 à 3, 6 et 7, ou procédé selon l'une quelconque des revendications 4, 6 et 7, dans lesquels le réseau de nanotubes comprend un côté supérieur et un côté inférieur, les deux desdits côtés comprenant les ouvertures des nanotubes.

11. Utilisation selon l'une quelconque des revendications 1 à 3 et 6 à 10, ou procédé selon l'une quelconque des revendications 4 et 6 à 10, dans lesquels le réseau de nanotubes peut être obtenu par anodisation électrolytique en utilisant une feuille métallique, sur laquelle un nanotube d'oxyde de métal est formé, en tant qu'électrode de travail.

12. Utilisation selon l'une quelconque des revendications 1 à 3 et 6 à 11, ou procédé selon l'une quelconque des revendications 4 et 6 à 11, dans lesquels le réseau de nanotubes comprend un revêtement de surface comprenant au moins une substance bioactive, de préférence sélectionnée parmi la poly-d-lysine, la poly-L-lysine, le collagène, la fibronectine et la laminine.
